(19) Europäisches Patentamt
European Patent Office
Office européen des brevets

(11) EP 2 274 056 B1

(12) **EUROPÄISCHE PATENTSCHRIFT**

(45) Veröffentlichungstag und Bekanntmachung des Hinweises auf die Patenterteilung:
**03.10.2012 Patentblatt 2012/40**

(21) Anmeldenummer: **09745592.7**

(22) Anmeldetag: **17.02.2009**

(51) Int Cl.:
***A61Q 5/06*** (2006.01) ***A61Q 5/08*** (2006.01)
***A61K 8/49*** (2006.01) ***A61K 8/22*** (2006.01)
***A61K 8/41*** (2006.01)

(86) Internationale Anmeldenummer:
**PCT/EP2009/051834**

(87) Internationale Veröffentlichungsnummer:
**WO 2009/138259 (19.11.2009 Gazette 2009/47)**

(54) **AUFHELLMITTEL MIT KATIONISCHEN 3,4-DIHYDROISOCHINOLINIUMDERIVATEN, SPEZIELLEN ALKANOLAMINEN UND WASSERSTOFFPEROXID**

BLEACHING AGENT COMPRISING CATIONIC 3,4-DIHYDROISOCHINOLINIUM DERIVATIVES, SPECIFIC ALKANOLAMINES AND HYDROGEN PEROXYDE

AGENT ÉCLAIRCISSANT CONTENANT DES DÉRIVÉS CATIONIQUES DE 3,4-DIHYDROISOQUINOLINIUM, DES ALCANOLAMINES SPÉCIFIQUES ET DU PEROXYDE D'HYDROGÈNE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **16.05.2008 DE 102008024030**

(43) Veröffentlichungstag der Anmeldung:
**19.01.2011 Patentblatt 2011/03**

(73) Patentinhaber: **Henkel AG & Co. KGaA**
**40589 Düsseldorf (DE)**

(72) Erfinder:
- **GROSS, Wibke**
  **41836 Hückelhoven (DE)**
- **NEMITZ, Ralph**
  **41363 Jüchen (DE)**
- **FUHR, Denise**
  **22559 Hamburg (DE)**
- **SENDOR, Dorota**
  **40589 Düsseldorf (DE)**
- **PAULI, Kristin**
  **42119 Wuppertal (DE)**
- **KNÜBEL, Georg**
  **40219 Düsseldorf (DE)**

(56) Entgegenhaltungen:
**EP-A- 1 905 418** **EP-B- 1 169 423**
**EP-B- 1 169 424** **WO-A-98/23717**
**WO-A-2005/120447** **US-A- 3 823 231**
**US-A- 5 576 282** **US-A- 5 785 886**

EP 2 274 056 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft Mittel zum Aufhellen keratinischer Fasern, d.h. Mittel zur Anwendung auf Keratinfasern, insbesondere menschlichen Haaren, enthaltend kationische 3,4-Dihydroisochinoliniumderivate, spezielle Alkanolamine und Wasserstoffperoxid, die Verwendung dieser Kombination zum Aufhellen von Haaren sowie ein entsprechendes Verfahren.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden. Dagegen sind Färbe- und Blondierverfahren auf bestimmte Ausgangshaarfarben begrenzt. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

Konventionelle Haarfärbemittel bestehen in der Regel aus mindestens einer Entwickler- und mindestens einer Kupplersubstanz und enthalten ggf. noch direktziehende Farbstoffe als Nuanceure. Kuppler- und Entwicklerkomponenten werden auch als Oxidationsfarbstoffvorprodukte bezeichnet.

Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Die in diesen Produkten enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalten eingesetzt. Mit der Aufhellung geht jedoch auch eine Schädigung des Haares einher, da nicht nur die natürlichen farbgebenden Komponenten des Haares, sondern auch die übrigen Strukturbestandteile des Haares oxidativ geschädigt werden. Je nach Ausprägung des Schädigungsgrades reicht dieser von rauhem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids und gegebenenfalls der Peroxodisulfate ist, desto stärkere Schädigungen werden in der Regel auf der Keratinfaser hervorgerufen. Haarfärbe- bzw. Aufhellmittel, welche eine gute Aufhellleistung zeigen, ohne gleichzeitig die Haarfaser zu schädigen, sind bislang nicht bekannt.

Vor ihrer Anwendung auf menschliches Haar werden Haarfärbe- und/oder -aufhellungsmittel in fester oder pastöser Form mit verdünnter wässriger Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Ausfärbung bzw. Aufhellung liegt zwischen etwa 30 und 40 Minuten. Es ist nahe liegend, dass bei den Benutzern dieser Haarfarben oder Blondiermittel ein Bedürfnis besteht, diese Einwirkungszeit zu verringern.

Blondierprozesse an keratinischen Fasern laufen üblicherweise bei alkalischen pH-Werten ab, insbesondere zwischen 9,0 und 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Als Alkalisierungsmittel wird üblicherweise Ammoniak eingesetzt, der allerdings für die Anwender den Nachteil des intensiven Geruches und eventueller Reizung aufweist.

Auch wenn die bislang auf dem Markt befindlichen Blondiermittel in der Regel gute Aufhellleistungen zeigen, so können sie aufgrund von Haarschädigung, langen Anwendungszeiten und der aufgrund der hohen Konzentrationen an Oxidations- und Alkalisierungsmittel möglichen Hautreizungen nicht als optimal angesehen werden.

Aus WO 2005/0120447 A1 sind bestimmte Imidazol-Verbindungen als Aktivatoren für Wasserstoffperoxid bekannt. Die EP1905418A1 offenbart Sechs-N-Heterocyclen zur Wasserstoffperoxid-Aktivierung. Keines dieser Dokumente offenbart irgendwelche kationischen Heterocyclen für die Aktivierung von Wasserstoffperoxid.

Aus EP 1169423 B1 und EP 116924 B1 sind mehrkomponentige Oberflächenreinigungsmittel mit bestimmten 3,4-Dihydroisochinolin-Verbindungen in Kombination mit Wasserstoffperoxid bekannt. Einen Hinweis auf die Verwendung auf keratinischen Fasern erhält der Fachmann aus diesen Dokumenten jedoch nicht.

Aus WO 98/23717 A1 sind N-Methyl-3,4-dihydroisochinolinium-Salze als Sauerstoff-Übertragungs-Verbindungen für die Verbesserung der Bleichkraft von Peroxoverbindungen in Reinigungsmitteln zur Fleckenbeseitigung bekannt. Jedoch ist die Anwendung auf keratinische Fasern nicht bekannt.

US 5,785,886A offenbart Bleichsysteme mit bestimmten Imin-Verbindungen und Übergangsmetallen zur Verbesserung der Bleichwirkung zur Fleckenentfemung. Aus US 5,576,282A sind farbechte Bleichkraftverstärker, insbesondere anionische und zwitterionische Dihydryoisochinolinium-Verbindungen, und deren Einsatz in Textilwaschmitteln bei niedrigen Temperaturen bekannt. Die Anwendung in Blondiermitteln für keratinische Fasern ist nicht offenbart.

Der Einsatz von kationischen Isochinoliniumderivaten als Bleichaktivatoren zur Optimierung der Bleichleistung von oxidativen Bleichwirkstoffen auf Haaren wird in der deutschen Patentanmeldung mit der Anmeldenummer 102007047688.6 (WO 2009 04 3613) beschrieben.

Es ist die Aufgabe dieser Erfindung, neuartige Mittel mit Bleichaktivator zum Aufhellen bzw. Blondieren von Haaren bereitzustellen, welche in ihrer Aufhellleistung dem bekannten Stand der Technik vergleichbar bzw. gegenüber diesem nach Möglichkeit überlegen sind, gleichzeitig jedoch eine gegenüber dem Stand der Technik als vergleichbar oder in erstrebenswerter Weise geringer einzustufende Haarschädigung bewirken.

Bekanntermaßen können kationische Isochinoliniumderivate in Kombination mit Imidazol die Bleichwirkung auf dem Haar optimieren. Der Einsatz von Imidazol in kosmetischen Mitteln bringt jedoch verschiedene Nachteile, speziell in toxikologischer Hinsicht, mit sich. Eine weitere Aufgabe dieser Erfindung ist es daher, für die erfindungsgemäßen Bleichaktivatoren einen dem Stand der Technik mindestens vergleichbaren Ersatz für Imidazol aufzufinden.

Es konnte nun in nicht vorhersehbarer Weise gefunden werden, daß der Einsatz einer Kombination von kationischen 3,4-Dihydroisochinoliniumverbindungen der allgemeinen Struktur (I), bestimmten Alkanolaminen der allgemeinen Struktur (II) und Wasserstoffperoxid die Haare viel stärker aufhellt, als es durch den Einsatz der kationischen 3,4-Dihydroisochinolinium-verbindungen und Wasserstoff allein bzw. durch deren Kombination mit Imidazol möglich wäre.

Demzufolge konnte überraschend gefunden werden, dass die durch den Einsatz der erfindungsgemäßen Kombination hervorgerufene Verbesserung der Aufhellung eine hervorragende Möglichkeit darstellt, das Imidazol zu ersetzen.

Bedingt durch die verbesserte Blondierleistung bei Anwendung des erfindungsgemäßen Mittels kann die Menge an eingesetztem Oxidationsmittel vermindert und die Haarschädigung hierdurch minimiert werden. Auch eine Verkürzung der Einwirkzeit unter Erzielung eines dem Stand der Technik entsprechenden Aufhelleffekts sind auf diese Weise möglich.

Ein erster Gegenstand der Erfindung ist daher ein Mittel zum Aufhellen von keratinischen Fasern, dadurch gekennzeichnet, dass es in einem kosmetischen Träger

- mindestens ein kationisches 3,4-Dihydroisochinoliniumderivat der nachfolgenden allgemeinen Struktur (I),

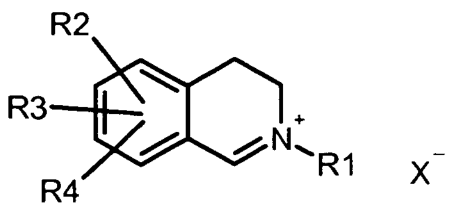

(I),

worin

| | |
|---|---|
| der Rest R1 | für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_1$-$C_6$-alkylgruppe, eine Aryl-$C_1$-$C_6$-alkylgruppe, eine $C_1$-$C_6$-Dialkylamino-$C_2$-$C_6$-alkylgruppe, eine Heteroaryl-$C_1$-$C_6$-alkylgruppe, eine 3-Oxobutylgruppe, eine 2-Oxopropylgruppe, eine Arylgruppe oder eine Heteroarylgruppe steht, |
| die Reste R2, R3 und R4 | unabhängig voneinander ein Wasserstoffatom, eine Hydroxygruppe, eine Aminogruppe, eine Di($C_1$-$C_6$)alkylaminogruppe, eine $C_1$-$C_6$-Alkoxygruppe, Halogen, eine Nitrogruppe, eine Carboxygruppe, eine Nitrilgruppe, eine gegebenenfalls substituierte Arylgruppe, eine $C_2$-$C_6$-Alkenylgruppe, eine gegebenenfalls substituierte Heteroarylgruppe darstellen können oder R2 und R3 zusammen einen weiteren ankondensierten carbozyklischen oder heterozyklischen Ring bilden können, welcher gesättigt oder ungesättigt sein kann und gegebenenfalls durch bis zu drei Substituenten substituiert sein kann, |

das Anion $X^-$ für ein physiologisch verträgliches Anion steht,

- mindestens ein Alkanolamin der nachfolgenden allgemeinen Struktur (II),

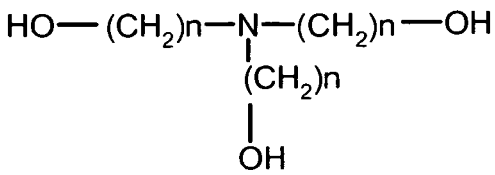

(II),

worin

n für eine natürliche Zahl von 2 bis 6 steht, und

- Wasserstoffperoxid enthält.

Unter Keratinfasern sind dabei Pelze, Wolle, Federn und insbesondere menschliche Haare zu verstehen. Obwohl die erfindungsgemäßen Mittel in erster Linie zum Färben und/oder Aufhellen von Keratinfasern geeignet sind, steht prinzipiell einer Verwendung auch auf anderen Gebieten nichts entgegen.

Im Folgenden werden Beispiele für die als Substituenten der Verbindungen der Formel (I) genannten Reste aufgezählt: Beispiele für ($C_1$ bis $C_6$)-Alkylreste sind die Gruppen $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$.

Erfindungsgemäße Beispiele für ($C_1$ bis $C_6$)-Alkoxyreste sind $-OCH_3$, $-OCH_2CH_3$, $-OCH_2CH_2CH_3$, $-OCH(CH_3)_2$, $-OCH_2CH_2CH_2CH_3$, $-OCH_2CH(CH_3)_2$, $-OCH(CH_3)CH_2CH_3$, $-OC(CH_3)_3$, insbesondere eine Methoxy- oder eine Ethoxygruppe.

Weiterhin können als bevorzugte Beispiele für eine ($C_2$ bis $C_6$)-Hydroxyalkylgruppe $-CH_2CH_2OH$, -$CH_2CH_2CH_2OH$, $-CHCH(OH)CH_3$, $-CH_2CH_2CH_2CH_2OH$, wobei die Gruppe $-CH_2CH_2OH$ bevorzugt ist.

Beispiele für Halogenatome sind F-, Cl- oder Br-Atome, Cl-Atome sind ganz besonders bevorzugte Beispiele.

Beispiele für ($C_1$ bis $C_4$)-Dialkylaminogruppe sind $-N(CH_3)_2$, $-N(CH_2CH_3)_2$.

Beispiele für ($C_1$ bis $C_4$)-Alkoxy-($C_1$ bis $C_4$)-alklgruppen sind die Gruppen $-CH_2CH_2OCH_3$, $-CH_2CH_2CH_2OCH_3$, $-CH_2CH_2OCH_2CH_3$, $-CH_2CH_2CH_2OCH_2CH_3$, $-CH_2CH_2OCH(CH_3)_2$, $-CH_2CH_2CH_2OCH(CH_3)_2$.

Beispiele für eine $C_2$-$C_6$-Alkenylgruppe sind eine 2-Propenylgruppe (Allylgruppe), eine But-3-enylgruppe, eine But-2-enylgruppe, eine Pent-4-enylgruppe oder eine Pent-3-enylgruppe. Die 2-Propenylgruppe ist in diesem Zusammenhang besonders bevorzugt.

Beispiele für eine Carboxy-$C_2$-$C_6$-alkylgruppe sind die Carboxymethylgruppe, die 2-Carboxyethylgruppe oder die 3-Carboxypropylgruppe.

Beispiele für eine Heteroaryl-$C_1$-$C_6$-alkylgruppe sind die Pyridin-2-ylmethylgruppe, die Pyridin-3-ylmethylgruppe, die Pyridin-4-ylmethylgruppe, die Pyrimidin-2-ylmethylgruppe, die 1H-Pyrrol-1-ylmethylgruppe, die 1H-Pyrool-1-ylethylgruppe, die 1H-Pyrazol-1-ylmethylgruppe oder die 1H-Pyrazol-1-ylethylgruppe.

Ein Beispiel für Arylgruppen ist die Phenylgruppe.

Beispiele für Aryl-($C_1$ bis $C_4$)-alkylgruppen sind die Benzylgruppe und die 2-Phenylethylgruppe.

Die erfindungsgemäßen Mittel enthalten mindestens drei wesentliche Bestandteile: mindestens ein kationisches 3,4-Dihydroisochinoliniumderivat der Formel (I), mindestens ein Alkanolamin der Formel (II) und Wasserstoffperoxid. Erfindungsgemäße Mittel können auch "Anwendungsmischungen" sein, d.h. Mittel, die zwar (z.B. aus Stabilitätsgründen) getrennt verpackt vorliegen, vor der Anwendung aber miteinander zu einer Anwendungsmischung vermischt und dann appliziert werden.

Bevorzugt ist es, wenn der Rest R1 der allgemeinen Struktur (I) für eine $C_1$-$C_6$-Alkylgruppe, für eine $C_2$-$C_6$-Alkenylgruppe oder für eine $C_2$-$C_6$-Hydroxyalkylgruppe steht.

Des Weiteren ist es erfindungsgemäß favorisiert, wenn die Reste R2, R3 und R4 der allgemeinen Struktur (I) ein Wasserstoffatom darstellen.

Es ist bevorzugt, wenn X- gemäß Formel (I) ausgewählt wird aus Halogenid (Chlorid, Bromid, Iodid), Benzolsulfonat, p-Toluolsulfonat, $C_1$-$C_4$-Alkansulfonat, Trifluormethansulfonat, Acetat, Triflluoracetat, Perchlorat, ½ Sulfat, Hydrogensulfat, Tetrafluoroborat, Hexafluorophosphat, Hexafluorozinkat oder Tetrafluorozinkat.

Erfindungsgemäß besonders begünstigt ist es, wenn das physiologisch verträgliche Anion $X^-$ für ein Halogenidion (insbesondere Chlorid oder Bromid), Hydrogensulfat, ½ Sulfat, p-Toluolsulfonat, Benzolfulfonat oder Acetat steht.

Besonders bevorzugte kationische 3,4-Dihydroisochinoliniumderivate der allgemeinen Formel (I) sind

| | |
|---|---|
| 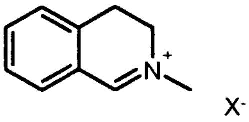 | 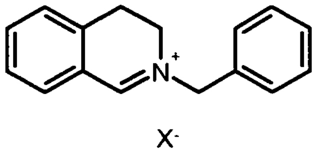 |
| Salze des N-Methyl-3,4-dihydroisochinoliniums | Salze des 3,4-Dihydro-2-(phenylmethyl)-isochinoliniums |
| 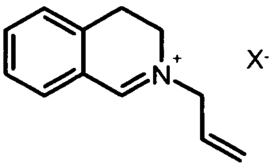 | 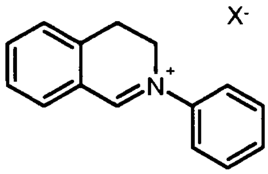 |
| Salze des N-Allyl-3,4-dihydroisochinoliniums | Salze des 3,4-Dihydro-2-phenyl-isochinoliniums |
| 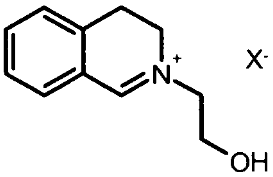 | 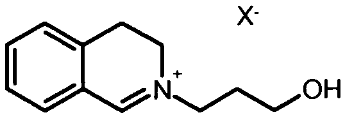 |
| Salze des N-(2-Hydroxyethyl)-3,4-dihydroisochinoliniums | Salze des 3,4-Dihydro-2-(3-hydroxypropyl)-isochinoliniums |
| 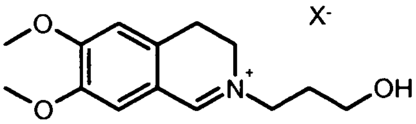 | 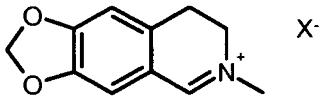 |
| Salze des 3,4-Dihydro-2-(3-hydroxypropyl)-6,7-dimethoxy-isochinoliniums | Salze des 7,8-Dihydro-6-methyl-1,3-dioxolo[4,5-g] isochinoliniums |
| 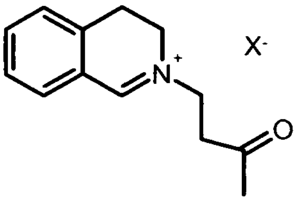 | 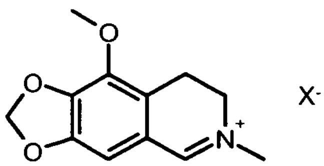 |
| Salze des 3,4-Dihydro-2-(3-oxobutyl)-isochinoliniums | Salze des 7,8Dihydro-4-methoxy-6-methyl-1,3-dioxolo [4,5-g]isochinoliniums |
| 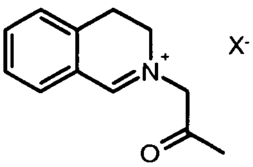 | 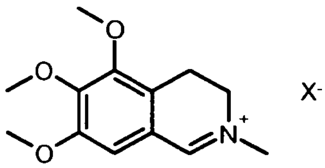 |
| Salze des 3,4-Dihydro-2-(2-oxopropyl)-isochinoliniums | Salze des 3,4-Dihydro-5,6,7-trimethoxy-2-methylisochinoliniums |
| 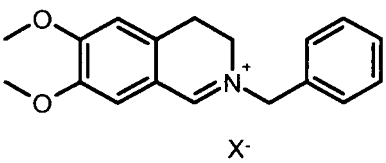 | 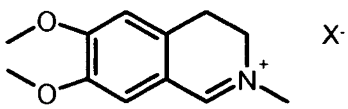 |
| Salze des 3,4-Dihydro-6,7-dimethoxy-2-(phenylmethyl) isochinoliniums | Salze des 3,4-Dihydro-6,7-dimethoxy-2-methylisochinoliniums |

worin $X^-$ jeweils die Bedeutungen gemäß Struktur (I), bzw. die Bedeutung der vorgenannten bevorzugten Ausführungsformen, annimmt.

Zusammenfassend sind erfindungsgemäße Mittel ganz besonders bevorzugt, die als kationisches 3,4-Dihydroisochinoliniumderivat der allgemeinen Struktur (I) mindestens eine Verbindung aus der Gruppe N-Methyl-3,4-dihydroisochinolinium-p-toluolsulfonat, N-Methyl-3,4-dihydroisochinoliniumbenzolsulfonat, N-Methyl-3,4-dihydroisochinoliniumhydrogensulfat, N-Allyl-3,4-dihydroisochinolinium-p-toluolsulfonat, N-Allyl-3,4-dihydroisochinoliniumbenzolsulfonat, N-Allyl-3,4-dihydroisochinoliniumbromid, N-Allyl-3,4-dihydroisochinoliniumacetat, 3,4-Dihydro-2-(3-hydroxypropyl) isochinolinium-p-toluolsulfonat, 3,4-Dihydro-2-(3-hydroxypropyl)isochinolinium-benzolsulfonat, 3,4-Dihydro-2-(3-hydroxypropyl)isochinoliniumbromid, 3,4-Dihydro-2-(3-hydroxypropyl)isochinoliniumacetat, 3,4-Dihydro-2-(2-hydroxyethyl) isochinolinium-p-toluolsulfonat, 3,4-Dihydro-2-(2-hydroxyethyl)isochinolinium-benzolsulfonat, 3,4-Dihydro-2-(2-hydroxyethyl)isochinoliniumbromid oder 3,4-Dihydro-2-(2-hydroxyethyl)isochinoliniumacetat enthalten.

Als Alkanolamine können verschiedene Trialkanolamine mit einer Kettenlänge von 2 bis 6 C-Atomen eingesetzt werden. Bevorzugt ist es, wenn n eine ganze Zahl von 2 bis 4 darstellt.

Insbesondere bevorzugt ist es, wenn es sich bei dem Alkanolamin der allgemeinen Formel (II) um Triethanolamin (Alternativname: Tris(2-hydroxyethyl)amin) handelt.

Soweit nicht explizit anders angegeben, beziehen sich nachfolgend genannte Mengenangaben jeweils auf das Gewicht des anwendungsbereiten Mittels.

Als ersten wesentlichen Inhaltstoff enthalten die erfindungsgemäßen Mittel die kationischen 3,4-Dihydroisochinoliniumderivate der allgemeinen Struktur (I) bevorzugt in einer Menge von 0,03 bis 65,00 mmol, insbesondere von 1,00 bis 40,00 mmol, jeweils bezogen auf 100 g des anwendungsbereiten Mittels.

Als zweiten wesentlichen Inhaltsstoff enthalten die erfindungsgemäßen Mittel die Alkanolamine der allgemeinen Formel (II) bevorzugt in einer Menge von 0,03 bis 65,00 mmol, insbesondere 1,00 bis 40,00 mmol, jeweils bezogen auf 100 g des anwendungsbereiten Mittels.

Als dritter wesentlicher Inhaltsstoff ist Wasserstoffperoxid in dem erfindungsgemäßen Mittel enthalten. Bevorzugt wird Wasserstoffperoxid selbst als wässrige Lösung verwendet. Das Wasserstoffperoxid kann aber auch in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon n $H_2O_2$ (n ist eine positive ganze Zahl größer 0), Harnstoffperoxid und Melaminperoxid, eingesetzt werden.

Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet. Erfindungsgemäß bevorzugte Mittel sind dadurch gekennzeichnet, dass sie - bezogen auf ihr Gewicht - 0,5 bis 12 Gew.-%, vorzugsweise 2 bis 10 Gew.-%, besonders bevorzugt 3 bis 6 Gew.-% Wasserstoffperoxid (berechnet als 100%iges $H_2O_2$) enthalten.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des verwendungsbereiten Mittels zwischen 7 und 11, insbesondere zwischen 8 und 10,5, liegt.Bei den pH-Werten im Sinne der vorliegenden Erfindung handelt es sich um pH-Werte, die bei einer Temperatur von 22 °C gemessen wurden.

Sollte der pH-Wert durch das Vorhandensein der erfindungsgemäßen Menge mindestens eines Alkanolamins der allgemeinen Struktur (II) noch nicht im gewünschten Bereich liegen, ist es möglich, weitere Alkalisierungsmittel aus der Gruppe, die gebildet wird aus Ammoniak, basischen Aminosäuren, Alkalihydroxiden, Alkalimetallmetasilikaten, Alkaliphosphaten und Alkalihydrogenphosphaten, zur Einstellung des gewünschten pH-Wertes einzusetzen. Als Alkalimetallionen dienen bevorzugt Lithium, Natrium, Kalium, insbesondere Natrium oder Kalium. Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Lysin, D-Lysin, D,L-Lysin, besonders bevorzugt L-Arginin, D-Arginin, D,L-Arginin als ein Alkalisierungsmittel im Sinne der Erfindung eingesetzt. Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Für die starke Aufhellung sehr dunklen Haares ist der Einsatz von Wasserstoffperoxid oder dessen Anlagerungsprodukten an organische bzw. anorganische Verbindungen oftmals nicht ausreichend. In diesen Fällen wird in der Regel eine Kombination aus Wasserstoffperoxid und Persulfaten eingesetzt. Es hat sich gezeigt, dass die Beimengung der erfindungsgemäßen 3,4-Dihydroisochinoliniumverbindungen der allgemeinen Formel (I) zusammen mit Alkanolaminen der Formel (II) nicht nur bei Wasserstoffperoxid allein, sondern auch bei einer Kombination aus Wasserstoffperoxid und Persulfatsalzen in einer Steigerung des Aufhellvermögens resultiert.

Daher kann es, sollte der Verbraucher den Wunsch nach einer sehr starken Blondierung verspüren, in einer weiteren Ausführungsform bevorzugt sein, wenn neben der kationischen 3,4-Dihydroisochinoliniumverbindung der allgemeinen Struktur (I), Alkanolaminen der Formel (II) und Wasserstoffperoxid zusätzlich mindestens ein anorganisches Persulfatsalz in dem Mittel zum Aufhellen der keratinischen Fasern enthalten ist.

Die Persulfatsalze können in einer Menge von 0,1 bis 25 g, insbesondere in einer Menge von 1 bis 15 g, bezogen auf 100 g des anwendungsbereiten Mittels, enthalten sein.

Bevorzugte Persulfatsalze sind Ammoniumperoxodisulfat, Kaliumperoxodisulfat und Natriumperoxodisulfat (Alternativnamen Ammoniumpersulfat, Kaliumpersulfat und Natriumpersulfat).

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden.

Ein üblicher Weg besteht daher darin, ein erstes Mittel, das die kationischen 3,4-Dihydroisochinoliniumverbindungen der allgemeinen Formel (I) sowie ein Alkanolamin der allgemeinen Formel (II) enthält, direkt vor der Anwendung mit einem zweiten Mittel, in welchem das oder die erfindungsgemäßen Oxidationsmittel enthalten sind, zu vermischen.

Ein weiterer Gegenstand der vorliegenden Erfindung ist daher ein Mittel zum Aufhellen keratinischer Fasern, insbesondere menschlicher Haare, welches unmittelbar vor dem Aufbringen auf das Haar aus einer fließfähigen Zubereitung A, das die kationischen 3,4-Dihydroisochinoliniumverbindungen der allgemeinen Formel (I) sowie ein Alkanolamin der allgemeinen Formel (II) enthält und einer Oxidationsmittelzubereitung B, enthaltend mindestens ein Oxidationsmittel, ausgewählt aus Wasserstoffperoxid und/oder dessen Anlagerungsverbindungen an organische oder anorganische Verbindungen, erhalten wird.

Die Oxidationsmittelzubereitung B ist vorzugsweise eine wäßrige, fließfähige Oxidationsmittelzubereitung. Dabei sind bevorzugte erfindungsgemäße Mittel zum Aufhellen keratinischer Fasern dadurch gekennzeichnet, daß die fließfähige Oxidationsmittelzubereitung B - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Der Einsatz von Persulfatsalzen erfolgt in der Regel in Form eines gegebenenfalls entstaubten Pulvers oder eines in Form gepressten Formkörpers. Um einen vorzeitigen Abbau der erfindungsgemäßen 3,4-Dihydroisochinoliniumderivate durch den Kontakt mit den Persulfaten zu vermeiden, ist es erfindungsgemäß bevorzugt, die Persulfate als separat verpackte Komponente C zur Verfügung zu stellen.

In diesem Zusammenhang ist ein aus 3 Komponenten bestehendes Mittel zum Aufhellen von menschlichen Haaren ein weiterer Gegenstand der vorliegenden Erfindung. Dieses Mittel wird unmittelbar vor dem Aufbringen auf das Haar durch sorgfältiges Vermischen einer fließfähigen Zubereitung A, die die kationischen 3,4-Dihydroisochinoliniumverbindungen der allgemeinen Formel (I) und ein Alkanolamin der allgemeinen Formel (II) enthält, einer Oxidationsmittelzubereitung B, enthaltend mindestens ein Oxidationsmittel, ausgewählt aus Wasserstoffperoxid und/oder dessen Anlagerungsverbindungen an organische oder anorganische Verbindungen und zusätzlich einer dritten, in Pulverform vorliegenden Zubereitung C, welche mindestens ein anorganisches Persulfatsalz enthält, hergestellt.

Die Mischung der Zubereitungen A und B bzw. gegebenenfalls der Zubereitungen A, B und C vor der Anwendung führt zu einer Anwendungsmischung, die ein erfindungsgemäßes Mittel mit den drei zwingenden Inhaltsstoffen ist.

Vorzugsweise wird den fließfähigen Zubereitungen A und/oder B ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind. Diese Stoffe werden weiter unten ausführlich beschrieben.

Weiterhin hat es sich als vorteilhaft erwiesen, wenn die erfindungsgemäßen Färbe- und/oder Aufhellmittel nichtionogene grenzflächenaktive Stoffe enthalten. Dabei sind solche grenzflächenaktive Stoffe, die einen HLB-Wert von 5,0 und größer aufweisen, bevorzugt.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.

Bevorzugte nichtionogene grenzflächenaktive Stoffe sind

- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen; bevorzugte Verbindungen dieser Klasse sind Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal®B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche Verbindung ist das Handelsprodukt Dehypon®LT 054, ein $C_{12-8}$-Fettalkoholol + 4,5 Ethylenoxid-butylether;

- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure;
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid;
- Polyglycerinester und alkoxylierte Polyglycerinester. Bevorzugte Verbindungen dieser Klasse sind beispielsweise Poly(3)glycerindiisostearat (Handelsprodukt: Lameform®TGI (Henkel)) und Poly(2)glycerinpolyhydroxy-stearat (Handelsprodukt: Dehymuls®PGPH (Henkel));
- Sorbitan-Fettsäureester und alkoxylierte Sorbitan-Fettsäureester wie beispielsweise Sorbitanmonolaurat und Sorbitanmonolaurat + 20 Ethylenoxid (EO);
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten (beispielsweise Nonylphenol + 4 EO, Nonylphenol + 9 EO, Octylphenol + 3 EO und Octylphenol + 8 EO).

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfindungsgemäße Mittel erwiesen, die nichtionogene grenzflächenaktive Substanzen in Mengen von 1 - 5 Gew.-% enthalten.

Weiterhin können die erfindungsgemäßen Aufhellmittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuresalze mit 10 bis 18 C-Atomen in der Alkylgruppe und bis zu 12 Glykolethergruppen im Molekül wie $C_{12}H_{25}(C_2H_4O)_6CH_2COONa$ sowie insbesondere Salze von gesättigten und speziell ungesättigten $C_8$-$C_{22}$-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor.

Als nichtionische Tenside eignen sich insbesondere C8-C22-Alkylmono- und oligoglycoside und deren ethoxylierte Analoga. Insbesondere die nichtethoxylierten Verbindungen haben sich als besonders geeignet erwiesen.

Beispiele für die in den erfindungsgemäßen Haaraufhellungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Färbe- und/oder Aufhellmittel weitere Wirk-, Hilfs- und Zusatzstoffe, wie beispielsweise nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane, kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol, zwitterionische und amphotere Polymere wie beispielsweise Acrylamidopropyl-trimethylammoniumchlorid/Acrylat-Copolymere und Octylacrylamid/Methylmethacrylat/tert-Butylaminoethylmethacrylat/2-Hydroxypropylmethacrylat-Copolymere, anionische

Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat /Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere, Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol, Strukturanten wie Maleinsäure und Milchsäure, haarkonditionierende Verbindungen wie Phospholipide, beispielsweise Sojalecithin, Ei-Lecitin und Kephaline, Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate, Parfümöle, faserstrukturverbessernde Wirkstoffe, Entschäumer wie Silikone, Farbstoffe zum Anfärben des Mittels, Antischuppenwirkstoffe wie Piroctone Olamine, Zink Omadine und Climbazol, Lichtschutzmittel, insbesondere derivatisierte Benzophenone, Zimtsäure-Derivate und Triazine, Wirkstoffe wie Allantoin, Pyrrolidoncarbonsäuren und deren Salze sowie Bisabolol, Vitamine, Provitamine und Vitaminvorstufen, insbesondere solche der Gruppen A, $B_3$, $B_5$, $B_6$, C, E, F und H, Pflanzenextrakte, Cholesterin, Konsistenzgeber wie Zuckerester, Polyolester oder Polyolalkylether, Fette und Wachse wie Walrat, Bienenwachs, Montanwachs und Paraffine, Fettsäurealkanolamide, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel, Pigmente, Stabilisierungsmittel für Wassserstoffperoxid und andere Oxidationsmittel, Treibmittel wie Propan-Butan-Gemische, $N_2O$, Dimethylether, $CO_2$ und Luft, Antioxidantien, enthalten

Die erfindungsgemäßen Mittel können die Inhaltsstoffe in einem geeigneten wäßrigen, alkoholischen oder wäßrig-alkoholischen Träger enthalten. Zum Zwecke der Haarbleiche sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, eine pulverförmige oder auch Tabletten-förmige Formulierung bereitzustellen, was für Färbe- und/oder Aufhellmittel bevorzugt ist.

Unter wäßrig-alkoholischen Lösungen sind im Sinne der vorliegenden Erfindung wäßrige Lösungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Bevorzugte erfindungsgemäße Mittel sind dadurch gekennzeichnet, daß sie zusätzlich ein nichtwässriges Lösungsmittel enthalten, wobei besonders bevorzugte erfindungsgemäße Mittel das Lösungsmittel in einer Konzentration von 0,1 - 30 Gewichtsprozent, bevorzugt in einer Konzentration von 1 - 20 Gewichtsprozent, ganz besonders bevorzugt in einer Konzentration von 2 - 10 Gewichtsprozent, jeweils bezogen auf das Mittel, enthalten.

In weiter bevorzugten erfindungsgemäßen Mitteln ist das Lösungsmittel ausgewählt aus Ethanol, n-Propanol, Isoropanol, n-Butanol, Propylenglykol, n- Butylenglykol, Glycerin, Diethylenglykolmonoethylether, Diethylenglykolmono-n- butylether , Phenoxyethanol und Benzylalkohol sowie ihren Mischungen.

Die erfindungsgemäßen Mittel können zusätzlich auch Farbstoffe und/oder Farbstoffvorprodukte enthalten und somit als Mittel bereitgestellt werden, die gleichzeitig aufhellend und färbend wirken. Solche Mittel werden nachfolgend als "Färbemittel", als "aufhellende Färbemittel" oder als "Färbe- und Aufhellmittel" bezeichnet.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprdukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder in situ geringe Mengen Wasserstoffperoxid erzeugen und auf diese Weise die Oxidation der Farbstoffvorprodukte biokatalytisch aktivieren. Besonders

geeignete Katalysatoren für die Oxidation der Farbstoffvorläufer sind die sogenannten 2-Elektronen-Oxidoreduktasen in Kombination mit den dafür spezifischen Substraten, Pyranose-Oxidase (mit z.B. D-Glucose oder Galactose), Glucose-Oxidase (mit D-Glucose), Glycerin-Oxidase (mit Glycerin), Pyruvat-Oxidase (mit Benztraubensäure oder deren Salzen), Alkohol-Oxidase (mit Alkohol wie MeOH, EtOH), Lactat-Oxidase (mit Milchsäure), Tyrosinase-Oxidase (mit (Tyrosin), Uricase (mit Harnsäure), Cholinoxidase (mit Cholin) und Aminosäure-Oxidase (mit Aminosäuren).

Bei einer Anwendung von Oxidationsmitteln wird das eigentliche Aufhell- und/oder Färbemittel zweckmäßigerweise unmittelbar vor der Anwendung durch Mischung der Zubereitung des Oxidationsmittels mit der Zubereitung, enthaltend die Verbindungen der Formel (I) und der Formel (II) und gegebenenfalls Farbstoffvorprodukte, hergestellt. Das dabei entstehende gebrauchsfertige Aufhell- und/oder Haarfärbepräparat sollte bevorzugt einen pH-Wert im Bereich von 6 bis 12 aufweisen. Besonders bevorzugt ist die Anwendung der Aufhell- und/oder Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Zusätzlich können die erfindungsgemäßen Mittel weitere Inhaltsstoffe enthalten. Ein Einsatz bestimmter Metallionen oder -komplexe kann beispielsweise bevorzugt sein, um intensive Färbungen zu erhalten. Hier sind erfindungsgemäße Mittel bevorzugt, die zusätzlich Cu-, Fe-, Mn-, Ru-Ionen oder Komplexe dieser Ionen enthalten.

Bevorzugte erfindungsgemäße Mittel enthalten zusätzlich Cu-, Fe-, Mn-, Co-, Ce-, V-, Ru-Ionen oder Komplexe dieser Ionen, wobei besonders bevorzugte Mittel 0,0001 bis 2,5 Gew.-%, vorzugsweise 0,001 bis 1 Gew.-% mindestens einer Verbindung aus der Gruppe Kupferchlorid ($CuCl_2$), Kupfersulfat ($CuSO_4$), Eisen(II)sulfat, Mangan(II)sulfat, Mangan (II)chlorid, Kobalt(II)-chlorid, Cersulfat, Cerchlorid, Vanadiumsulfat, Mangandioxid ($MnO_2$) enthalten.

Erfindungsgemäß bevorzugte Mittel sind dadurch gekennzeichnet, daß sie zusätzlich einen oder mehrere Chelatkomplexbildner aus den Gruppen der

(i) Polycarbonsäuren, bei denen die Summe der Carboxyl- und gegebenenfalls Hydroxylgruppen mindestens 5 beträgt (insbesondere EDTA sowie deren Salze),
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine

enthalten, wobei bevorzugte Mittel Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, enthalten.

Wie bereits erwähnt, können die erfindungsgemäßen Mittel nicht nur als reine Aufhellmittel, d.h. als sogenannte Blondiermittel, sondern auch als Färbe- und Aufhellmittel bereitgestellt werden, die gleichzeitig mit der Aufhellung auch eine Färbung der Keratinfasern bewirken. Zu diesem Zweck enthalten solche erfindungsgemäßen Mittel mindestens ein Farbstoffvorprodukt, vorzugsweise ein Oxidationsfarbstoffvorprodukt und/oder mindestens einen direktziehenden Farbstoff.

Die erfindungsgemäßen Mittel dieser Ausführungsform sind neben ihrer Funktion als Blondiermittel auch Färbemittel, d.h. Mittel zur Veränderung der Farbe keratinischer Fasern. Unter diesen sind insbesondere die sogenannten Oxidationsfärbemittel bevorzugt. Die erfindungsgemäßen Oxidationsfärbemittel enthalten mindestens eine Kuppler- und mindestens eine Entwicklerkomponente. Kuppler- und Entwicklerkomponenten werden auch als Oxidationsfarbstoffvorprodukte bezeichnet. Zudem können die erfindungsgemäßen Oxidationsfärbemittel auch noch direktziehende Farbstoffe als Nuanceure enthalten.

Erfindungsgemäß bevorzugte Mittel zum Färben und/oder Aufhellen keratinischer Fasern sind demnach dadurch gekennzeichnet, daß sie mindestens ein Oxidationsfarbstoffvorprodukt vom Entwicklertyp und/oder Kupplertyp enthalten.

Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

In einer Ausführungsform zur Farbveränderung ist der Gegenstand der vorliegenden Erfindung mit mindestens einer farbverändernden Komponente kombinierbar. Die farbverändernden Komponenten im Sinne der vorliegenden Erfindung werden bevorzugt ausgewählt aus mindestens einem Oxidationsfarbstoffvorprodukt vom Typ der Entwicklerkomponenten und gegebenenfalls zusätzlich mindestens einer Kupplerkomponente und/oder aus mindestens einem direktziehenden Farbstoff.

Bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)propan-2-ol, N,N'-Bis-(4-aminophenyl)-1,4-diaza-cycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)phenol, 4,5-Diamino-1-(β-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy) ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis-(2'-hydroxyethylaminoyl-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)-amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Entwickler - und Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehenden Farbstoffe sind dabei kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die Verbindungen, die unter den Bezeichnungen Basic Yellow 87, Basic Orange 31 und Basic Red 51 bekannt sind, sind ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)-amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Ein zweiter Gegenstand der vorliegenden Erfindung ist ein Verfahren zum Aufhellen von Keratinfasern, insbesondere menschlichen Haaren, dadurch gekennzeichnet, dass ein Mittel des ersten Erfindungsgegenstandes auf die keratinhaltigen Fasern aufgebracht, 5 bis 60 Minuten auf der Faser belassen und anschließend wieder ausgespült oder mit einem Shampoo ausgewaschen wird. Insbesondere liegt die Temperatur während der Einwirkzeit von 5 bis 60 Minuten zwischen 10°C und 40°C, insbesondere zwischen 20°C und 38°C.

Im Rahmen eines solchen Verfahren kann es bevorzugt sein, das Verfahren dadurch zu kennzeichnen, dass gewünschtenfalls ein Vorbehandlungsmittel M1 auf die Faser aufgebracht wird, dann ein Mittel M2 auf der Faser zur Anwendung kommt, wobei gewünschtenfalls dem Mittel M2 vor der Anwendung ein weiteres Mittel M3 zugegeben wird, dieses Mittel M2 nach einer Zeit von 5-60 Minuten von der Faser abgespült wird und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 oder die Mischung der Mittel M2 und M3 ein erfindungsgemäßes Mittel des ersten Erfindungsgegenstandes ist.

Die erfindungsgemäßen Mittel können demnach als Einkomponentenmittel (Färbe- und/oder Aufhellmittel M2), als Zweikomponenten Mittel (M2 + M3) formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Ein Färbe- und/oder Aufhellungsverfahren, bei dem die Verbindungen der allgemeinen Struktur (I) sowie Alkanolamine der allgemeinen Struktur (II) und das Wasserstoffperoxid zunächst getrennt vorliegen, ist dabei bevorzugt. Ein weiterer Gegenstand der vorliegenden Erfindung ist daher ein Verfahren zum Färben und Aufhellen von menschlichen Haaren, bei dem eine Zusammensetzung auf wäßriger Grundlage, enthaltend Wasserstoffperoxid, mit einer Zusammensetzung, enthaltend mindestens eine Verbindung der allgemeinen Struktur (I) sowie Alkanolamine der allgemeinen Struktur (II) (*vide supra*) zu einem Mittel der ersten Erfindungsgegenstandes vermischt, und dieses auf das Haar aufgebracht wird.

In einer weiteren Ausführungsform des erfindungsgemäßen Verfahrens zum Aufhellen und gegebenenfalls Färben von menschlichen Haaren kann eine Zusammensetzung auf wäßriger Grundlage, enthaltend Wasserstoffperoxid, mit einem weiteren Mittel enthaltend vorzugsweise mindestens einen Alkalitätsgeber und/oder direktziehenden Haarfarbstoff und/oder mindestens ein Oxidationsfarbstoffvorprodukt, und einem Mittel enthaltend die Verbindungen der allgemeinen Struktur (I) (*vide supra*) und der Formel (II) (*vide supra*) zu einer homogenen Zusammensetzung vermischt und diese auf das Haar aufgebracht werden.

Ein dritter Gegenstand der Erfindung ist die Verwendung der Mittel des ersten Erfindungsgegenstandes zum Aufhellen von keratinhaltigen Fasern, insbesondere menschlichen Haaren.

Bezüglich weiterer bevorzugter Ausführungsformen der erfindungsgemäßen Verfahren bzw. der erfindungsgemäßen Verwendung gilt mutatis mutandis das zu den erfindungsgemäßen Mitteln Gesagte.

Beispiele

1.0 Synthesebeispiel

1.1. Synthese von N-(2-Phenylethyl)formamid

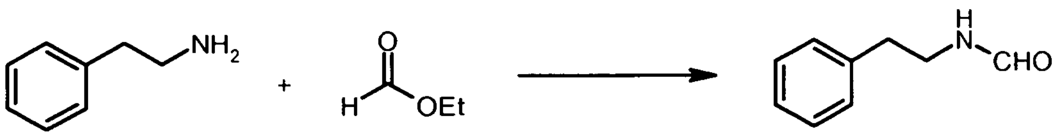

Es wurden 100,0 g (0,83 mol) Phenylethylamin und 187,0 g (2,07 mol) Ameisensäureethylester zusammmen für 12 Stunden unter Rückfluß erhitzt. Der überschüssige Ameisensäureethylester wurde im Vakuum am Roationsverdampfer entfernt. Als Rückstand verblieb ein nahezu farbloses Öl, welches ohne weitere Aufreinigung in der nächsten Stufe eingesetzt wurde; Ausbeute: 122,2 g (99,3 %); $^{1}H$-NMR (400 MHz, DMSO-$d_6$): δ [ppm] = 2,72 (t, 2H); 3,45 (t, 2H); 7,19 - 7,31 (m, 5H); 8,00 (s, 1H); 8,10 (br, NH); $^{13}C$-NMR (400 MHz, DMSO-d6): δ [ppm] = 35,0; 38,7; 125,9; 128,0; 128,2; 139,1; 160,1.

1.2. Synthese von 3,4-Dihydroisochinolin

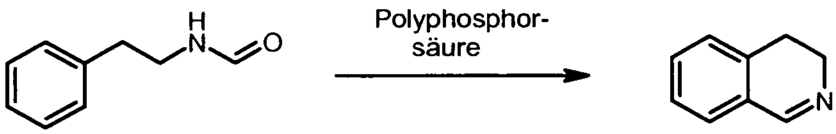


Es wurden 490,0 g (5,00 mol) Polyphsophorsäure auf 80 °C erhitzt, bis sie mit einem Metallrührer gut zu durchmischen war. Dann wurden unter Rühren bei 80 °C 84,0 g (0,56 mol) N-(2-Phenylethyl)formamid aus Stufe 1 zugegeben und das Gemisch für 12 Stunden auf 160 °C erhitzt. Nach der Reaktion wurde das Gemisch auf 1000 ml Eiswasser gegeben, dann wurde für 2 Stunden bei Raumtemperatur gerührt. Mit einer 5 molaren, wässrigen Natronlaugelösung wurde ein pH-Wert von 12,0 eingestellt. Die wässrige Phase wurde mit Methyl-*tert*-butylether extrahiert. Die vereinigten organischen Phasen wurden mit Magnesiumsulfat getrocknet und am Rotationsverdampfer komplett eingeengt, wobei ein dunkelbraunes Öl resultierte. Das Öl wurde im Vakuum destilliert (40 mbar/ 115 °C) und fiel in Form einer klaren, hellbraunen Flüssigkeit an, welche in der dritten Stufe eingesetzt wurde. Ausbeute: 56,3 g (76,1 %); $^{1}H$-NMR (400 MHz, DMSO-$d_6$): δ [ppm] = 2,68 (t, 2H); 3,65 (t, 2H); 7,23 (d, 1H); 7,34 (m, 2H); 7,41 (d, 1H); 8,34 (s, 1H); $^{13}C$-NMR (400 MHz, DMSO-d6): δ [ppm] = 25,4; 48,0; 127,5; 127,8; 128,9; 131,5; 137,0; 160,7.

1.3. Synthese von N-Methyl-3,4-dihydroisochinolinium-p-toluolsulfonat (A1)

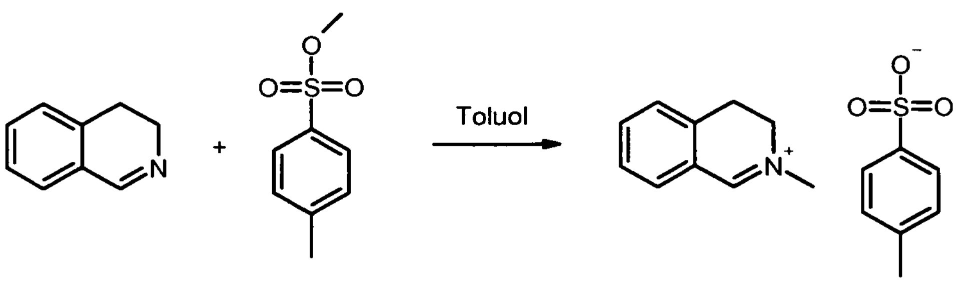


Es wurden 56,0 g (0,43 mol) 3,4-Dihydroisochinolin aus Stufe 2 zu einer Lösung von 80,0 g (0,43 mol) p-Toluolsulfonsäuremethylester in 250 ml Toluol gegeben. Das Reaktionsgemisch wurde für drei Stunden bei 60°C gerührt,

wobei die Lösung sich langsam trübte. Der nach dem Abkühlen ausgefallene Feststoff wurde abfiltriert und im Vakuum getrocknet. Ausbeute: 125,6 g (92,7 %); $^{1}$H-NMR (400 MHz, DMSO-$d_6$): $\delta$ [ppm] = 2,25 (s, 3H); 3,18 (t, 2H); 3,72 (s, 3H); 4,01 (t, 3H); 7,09 (d, 2H); 7,20 (m, 2H); 7,52 (d, 2H); 7,58 (m, 1H); 7,79 (m, 1H); 9,23 (s, 1H); $^{13}$C-NMR (400 MHz, DMSO-d6): $\delta$ [ppm] = 24,2; 26,8; 50,0; 52,7; 126,0; 127,3; 130,6; 130,8; 132,3; 136,0; 139,1; 140,1; 140,7; 148,1; 169,1

2. Beispiele zur Blondierung

2.1. Blondierungen mit Wasserstoffperoxid

2.1.1. Herstellung einer Blondiercreme

Aus den aufgelisteten Bestandteilen wurden Blondiercremes wie folgt hergestellt:

| Rohstoff | Gew.-% | | | | |
|---|---|---|---|---|---|
| | V1 | V2 | V3 | V4 | E |
| Hydrenol D | 6,9 | 6,9 | 6,9 | 6,9 | 6,9 |
| Lorol techn. | 2,5 | 2,5 | 2,5 | 2,5 | 2,5 |
| Eumulgin B1 | 0,6 | 0,6 | 0,6 | 0,6 | 0,6 |
| Eumulgin B2 | 0,6 | 0,6 | 0,6 | 0,6 | 0,6 |
| Alkypo Soft 45 NV | 10,0 | 10,0 | 10,0 | 10,0 | 10,0 |
| Plantacare 1200 UP | 2,0 | 2,0 | 2,0 | 2,0 | 2,0 |
| Texapon K 14 S 70 C | 2,8 | 2,8 | 2,8 | 2,8 | 2,8 |
| Ammoniumsulfat | 1,0 | 1,0 | 1,0 | 1,0 | 1,0 |
| Ascorbinsäure | 0,1 | 0,1 | 0,1 | 0,1 | 0,1 |
| Natriumsilikat 40/42 | 0,5 | 0,5 | 0,5 | 0,5 | 0,5 |
| Turpinal SL | 0,2 | 0,2 | 0,2 | 0,2 | 0,2 |
| Kaliumhydroxid | 0,8 | 0,8 | 0,8 | 0,8 | 0,8 |
| Ammoniak 25 % | 7,1 | 7,1 | 7,1 | 7,1 | 7,1 |
| Imidazol | - | - | 2,0 | - | - |
| Monoethanolamin | - | - | - | 2,0 | - |
| Triethanolamin | - | - | - | - | 2,0 |
| N-Methyl-3,4-dihydroisochinolinium-p-toluolsulfonat (A1) | - | 2,0 | 2,0 | 2,0 | 2,0 |
| Wasser | ad 100 | ad 100 | ad 100 | ad 100 | ad 100 |

Hydrenol® D $C_{16-18}$-Fettalkohol (INCI-Bezeichnung: Cetearyl alcohol) (Cognis)
Lorol® tech. $C_{12-18}$-Fettalkohol (INCI-Bezeichnung: Coconut alcohol) (Cognis)
Eumulgin® B 1 Cetylstearylalkohol mit ca. 12-EO-Einheiten (INCI-Bezeichnung: Ceteareth-12) (Cognis)
Eumulgin® B2 Cetylstearylalkohol mit ca. 20 EO-Einheiten (INCI-Bezeichnung: Ceteareth-20) (Cognis)
Akypo Soft 45 NV® Laurylalkohol-4.5-EO-Essigsäure-Natrium-Salz (mind. 21% Aktivsubstanzgehalt; INCI-Bezeichnung: Sodium Laureth-6 Carboxylate) (Chem-Y)
Plantacare® 1200 UP $C_{12-16}$-Fettalkohol-1.4-glucosid (ca. 50-53% Aktivsubstanzgehalt; INCI-Bezeichnung: Lauryl Glucoside, Aqua (Water)) (Cognis)
Texapon® K 14 S 70 C Laurylmyristylethersulfat-Natrium-Salz (ca. 68% bis 73% Aktivsubstanzgehalt'; INCI-Bezeichnung: Sodium Myreth Sulfate) (Cognis)
Natriumsilikat 40/42 Natronwasserglas
Turpinal® SL 1-Hydroxyethan-1,1-diphosphonsäure (ca. 58 - 61% Aktivsubstanzgehalt; INCI-Bezeichnung: Etidronic Acid, Aqua (Water)) (Solutia)

Hydrenol und Lorol wurden in der Wärme vordispergiert. Anschließend wurden die anderen Komponenten

unter Rühren der Reihe nach eingearbeitet, dann wurde mit Wasser auf 100 % aufgefüllt.

Bei den Rezepturen V1, V2, V3 und V4 handelt es sich um nicht erfindungsgemäße Vergleichsrezepturen. Rezeptur V1 stellt die eine Standardrezeptur ohne Bleichaktivator, die Rezeptur V2 eine Standardrezeptur mit Bleichaktivator dar. Bei den Rezepturen V3 und V4 handelt es sich um Formulierungen mit Bleichaktivator in Kombination mit Imidazol bzw. Monoethanolamin (2-Aminoethanol). Die Rezeptur E steht beispielhaft für eine erfindungsgemäße Zusammensetzung.

2.1.2. Vermischen mit der Entwicklerdisperion

Jede Blondiercreme wurde im Verhältnis 1:1 mit einer wie folgt zusammengesetzten Entwicklerdispersion ausgemischt. Der pH-Wert der fertigen Anwendungsmischung lag zwischen 9 und 10,2.

| Rohstoff | Gew.-% |
|---|---|
| Ammoniak 25 % | 0,62 |
| Dipicolinsäure | 0,10 |
| Dinatriumpyrophosphat | 0,03 |
| Turpinal SL | 1,50 |
| Texapon NSO | 2,00 |
| Dow Corning DB 110 A (nicht ionische Silikonemulsion) | 0,07 |
| Aculyn 33A (Acrylpolymer) | 12,00 |
| Wasserstoffperoxid 50 % | 22,40 |
| Wasser | ad 100 |
| Turpinal® SL 1-Hydroxyethan-1,1-diphosphonsäure (ca. 58 - 61% Aktivsubstanzgehalt; INCI-Bezeichnung: Etidronic Acid, Aqua (Water)) (Solutia)<br>Texapon® NSO Laurylethersulfat, Natriumsalz (ca. 27,5% Aktivsubstanz; INCI-Bezeichnung: Sodium Laureth Sulfate) (Cognis)<br>Aculyn® 33 Acrylpolymer (ca. 28% Festkörper in Wasser; INCI-Bezeichnung: Acrylates Copolymer) | |

Für den Blondierprozeß wurde auf Strähnen dunkelblonden, hellbraunen und dunkelbraunen Haares (Codes: Kerling 7/0, Fischbach & Miller 6923 und Kerling 2/0) von ca. 0,7 g Gewicht die 4-fache Menge der fertigen Anwendungsmischung appliziert. Nachdem die Strähnen für 30 Minuten bei 32 °C blondiert wurden, wurden sie mit einem handelsüblichen Shampoo gewaschen und mit einem Föhn getrocknet.

2.1.3. Auswertung der Aufhelleistung

Jede Haarsträhne wurde vor und nach dem Bleichvorgang farbmetrisch vermessen. Als Maß für die Aufhelleistung der jeweiligen Rezeptur wurde der dL-Wert gemäß folgender Formel herangezogen:

$$dL = L_{nachher} - L_{vorher}$$

$L_{nachher}$ = Helligkeit der Strähne nach dem Bleichen; $L_{vorher}$ = Helligkeit der Strähne vor dem Bleichen.

Für jede Rezeptur und jeden Haartyp erfolgte eine Doppelbestimmung, aus den Einzelwerten wurde jeweils der Mittelwert gebildet. Je größer der dL-Wert ist, desto besser ist die Aufhellleistung der jeweiligen Rezeptur.

Aufhelleistung bei dunkelblonden Strähnen (Kerling 7/0)

| dL (Rezeptur V1) | dL (Rezeptur V2) | dL (Rezeptur V3) | dL (Rezeptur V4) | dL (Rezeptur E) |
|---|---|---|---|---|
| 8,56 | 12,78 | 13,57 | 13,29 | 14,22 |

Aufhelleistung bei hellbraunen Strähnen (Fischbach & Miller 6923)

| dL (Rezeptur V1) | dL (Rezeptur V2) | dL (Rezeptur V3) | dL (Rezeptur V4) | dL (Rezeptur E) |
|---|---|---|---|---|
| 11,35 | 12,82 | 15,84 | 12,82 | 16,41 |

Aufhelleistung bei dunkelbraunen Strähnen (Kerling 2/0)

| dL (Rezeptur V1) | dL (Rezeptur V2) | dL (Rezeptur V3) | dL (Rezeptur V4) | dL (Rezeptur E) |
|---|---|---|---|---|
| 5,16 | 6,52 | 8,39 | 7,54 | 9,46 |

3. Deutung der Ergebnisse

Eine Abschätzung der Bleichwirkungen der unterschiedlichen Rezepturen läßt sich durch den Vergleich der dL-Werte treffen. Gegenüber der normalen Standardblondierung ohne Bleichaktivator (V1) kann durch Zusatz eines Bleichaktivators (V2) die Blondierleistung verbessert werden. Anhand der dL-Werte ist eindeutig erkennbar, dass das Aufhellergebnis durch Zusatz eines Wirkstoffs noch weiter verbessert werden kann (V3, V4 und E). Von allen eingesetzten Wirkstoffen zeigen die erfindungsgemäßen Alkanolamine - exemplarisch dargestellt am eingesetzten Triethanolamin (Rezeptur E) - durchgängig über alle untersuchten Haarsorten das beste Ergebnis mit den größten dL-Werten.

**Patentansprüche**

**1.** Mittel zum Aufhellen von keratinischen Fasern, **dadurch gekennzeichnet, dass** es in einem kosmetischen Träger

- mindestens ein kationisches 3,4-Dihydroisochinoliniumderivat der nachfolgenden allgemeinen Struktur (I),

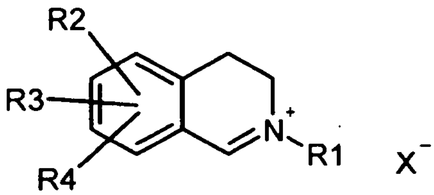

(I),

worin

der Rest R1 für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_1$-$C_6$-alkylgruppe, eine Aryl-$C_1$-$C_6$-alkylgruppe, eine $C_1$-$C_6$-Dialkylamino-$C_2$-$C_6$-alkylgruppe, eine Heteroaryl-$C_1$-$C_6$-alkylgruppe, eine 3-Oxobutylgruppe, eine 2-Oxopropylgruppe, eine Arylgruppe oder eine Heteroarylgruppe steht,
die Reste R2, R3 und R4 unabhängig voneinander ein Wasserstoffatom, eine Hydroxygruppe, eine Aminogruppe, eine Di($C_1$-$C_6$)alkylaminogruppe, eine $C_1$-$C_6$-Alkoxygruppe, Halogen, eine Nitrogruppe, eine Carboxygruppe, eine Nitrilgruppe, eine gegebenenfalls substituierte Arylgruppe, eine $C_2$-$C_6$-Alkenylgruppe, eine gegebenenfalls substituierte Heteroarylgruppe darstellen können oder R2 und R3 zusammen einen weiteren ankondensierten carbozyklischen oder heterozyklischen Ring bilden können, welcher gesättigt oder ungesättigt sein kann und gegebenenfalls durch bis zu drei Substituenten substituiert sein kann,

das Anion $X^-$ für ein physiologisch verträgliches Anion steht,
- mindestens ein Alkanolamin der nachfolgenden allgemeinen Struktur (II),

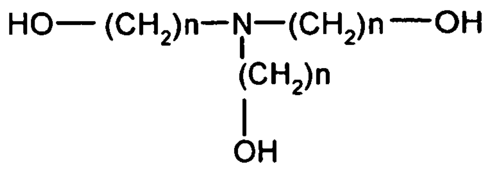

(II),

worin

n für eine natürliche Zahl von 2 bis 6 steht, und

- Wasserstoffperoxid enthält.

2. Mittel nach Anspruch 1, **dadurch gekennzeichnet, dass** der Rest R1 der allgemeinen Struktur (I) für eine $C_1$-$C_6$-Alkylgruppe, für eine $C_2$-$C_6$-Alkenylgruppe oder für eine $C_2$-$C_6$-Hydroxyalkylgruppe steht.

3. Mittel nach den Ansprüchen 1 und 2, **dadurch gekennzeichnet, dass** die Reste R2, R3 und R4 der allgemeinen Struktur (I) ein Wasserstoffatom darstellen.

4. Mittel nach einem der Ansprüche 1 bis 3, **dadurch gekennzeichnet, dass** das physiologisch verträgliche Anion $X^-$ für ein Halogenidion, Hydrogensulfat, ½ Sulfat, p-Toluolsulfonat, Benzolfulfonat oder Acetat steht.

5. Mittel nach einem der Ansprüche 1 bis 4, **dadurch gekennzeichnet, dass** mindestens eine kationische Verbindung der allgemeinen Struktur (I) ausgewählt aus Verbindungen der Gruppe, die gebildet wird aus
N-Methyl-3,4-dihydroisochinolinium-p-toluolsulfonat,
N-Methyl-3,4-dihydroisochinoliniumbenzolsulfonat
N-Methyl-3,4-dihydroisochinoliniumhydrogensulfat
N-Allyl-3,4-dihydroisochinolinium-p-toluolsulfonat
N-Allyl-3,4-dihydroisochinoliniumbenzolsulfonat
N-Allyl-3,4-dihydroisochinoliniumbromid
N-Allyl-3,4-dihydroisochinoliniumacetat
3,4-Dihydro-2-(3-hydroxypropyl)isochinolinium-p-toluolsulfonat
3,4-Dihydro-2-(3-hydroxypropyl)isochinolinium-benzolsulfonat
3,4-Dihydro-2-(3-hydroxypropyl)isochinoliniumbromid
3,4-Dihydro-2-(3-hydroxypropyl)isochinoliniumacetat
3,4-Dihydro-2-(2-hydroxyethyl)isochinolinium-p-toluolsulfonat
3,4-Dihydro-2-(2-hydroxyethyl)isochinolinium-benzolsulfonat
3,4-Dihydro-2-(2-hydroxyethyl)isochinoliniumbromid oder
3,4-Dihydro-2-(2-hydroxyethyl)isochinoliniumacetat
enthalten ist.

6. Mittel nach einem der Ansprüche 1 bis 5, **dadurch gekennzeichnet, dass** n in den Alkanolaminen der allgemeinen Formel (II) eine ganze Zahl von 2 bis 4 darstellt.

7. Mittel nach einem der Ansprüche 1 bis 6, **dadurch gekennzeichnet, dass** es sich bei dem Alkanolamin der allgemeinen Formel (II) um Triethanolamin handelt.

8. Mittel nach einem der Ansprüche 1 bis 7, **dadurch gekennzeichnet, dass** die kationischen 3,4-Dihydroisocholiniumderivate der allgemeinen Struktur (I) in einer Menge von 0,03 bis 65,00 mmol, insbesondere 1,00 bis 40,00 mmol, jeweils bezogen auf 100 g des anwendungsbereiten Mittels, enthalten sind.

9. Mittel nach einem der Ansprüche 1 bis 8, **dadurch gekennzeichnet, dass** die Alkanolamine der allgemeinen Struktur (II) in einer Menge von 0,03 bis 65,00 mmol, insbesondere 1,00 bis 40,00 mmol, jeweils bezogen auf 100 g des anwendungsbereiten Mittels, enthalten sind.

10. Mittel nach einem der Ansprüche 1 bis 9, **dadurch gekennzeichnet, dass** es - bezogen auf dessen Gewicht - 0,5 bis 12 Gew.-%, vorzugsweise 2 bis 10 Gew.-% und insbesondere bevorzugt 3 bis 6 Gew.-% Wasserstoffperoxid

(berechnet als 100 %iges $H_2O_2$) enthält.

**11.** Mittel nach einem der Ansprüche 1 bis 10, **dadurch gekennzeichnet**, das der pH-Wert der verwendungsbereiten Anwendungsmischung zwischen 7 und 11, insbesondere zwischen 8 und 10,5, liegt.

**12.** Mittel nach einem der Ansprüche 1 bis 11, **dadurch gekennzeichnet, dass** zusätzlich mindestens ein anorganisches Persulfatsalz enthalten ist.

**13.** Mittel nach Anspruch 12, **dadurch gekennzeichnet, dass** die anorganischen Persulfatsalze ausgewählt werden aus mindestens einer Verbindung aus der Gruppe bestehend aus Ammoniumperoxidisulfat, Kaliumperoxidisulfat und Natriumperoxidisulfat.

**14.** Verfahren zum Aufhellen von keratinischen Fasern, **dadurch gekennzeichnet, dass** ein Mittel nach einem der Ansprüche 1 bis 13 auf die keratinhaltigen Fasern aufgebracht, 5 bis 60 Minuten auf der Faser belassen und anschließend wieder ausgespült oder mit einem Shampoo ausgewaschen wird.

**15.** Kosmetische Verwendung eines Mittels nach einem der Ansprüche 1 bis 13 zum Aufhellen von keratinhalten Fasern, insbesondere menschlichen Haaren.

## Claims

**1.** An agent for bleaching keratin fibers, **characterised in that** it contains in a cosmetic carrier

- at least one cationic 3,4-dihydroisoquinoline derivative of the following general structure (I),

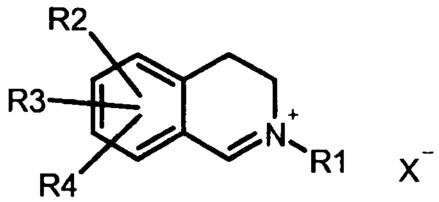

(I),

in which

the residue R1 denotes a $C_1$-$C_6$ alkyl group, a $C_2$-$C_6$ alkenyl group, a $C_2$-$C_6$ hydroxyalkyl group, a $C_1$-$C_6$ alkoxy $C_2$-$C_6$ alkyl group, a carboxy $C_1$-$C_6$ alkyl group, an aryl $C_1$-$C_6$ alkyl group, a $C_1$-$C_6$ dialkylamino $C_2$-$C_6$ alkyl group, a heteroaryl $C_1$-$C_6$ alkyl group, a 3-oxobutyl group, a 2-oxopropyl group, an aryl group or a heteroaryl group,
the residues R2, R3 and R4 independently of one another can denote a hydrogen atom, a hydroxyl group, an amino group, a di($C_1$-$C_6$)alkylamino group, a $C_1$-$C_6$ alkoxy group, halogen, a nitro group, a carboxy group, a nitrile group, an optionally substituted aryl group, a $C_2$-$C_6$ alkenyl group, an optionally substituted heteroaryl group or R2 and R3 together can form a further fused carbocyclic or heterocyclic ring, which can be saturated or unsaturated and can optionally be substituted by up to three substituents,
the anion $X^-$ denotes a physiologically tolerable anion,

- at least one alkanol amine of the following general structure (II),

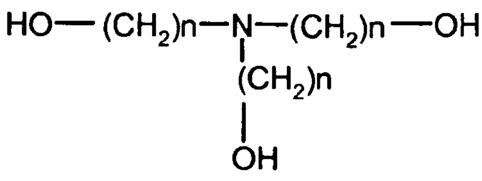

(II),

in which

n denotes a natural number from 2 to 6, and

- hydrogen peroxide.

2. The agent according to claim 1, **characterised in that** the residue R1 of the general structure (I) denotes a $C_1$-$C_6$ alkyl group, a $C_2$-$C_6$ alkenyl group or a $C_2$-$C_6$ hydroxyalkyl group.

3. The agent according to claims 1 and 2, **characterised in that** the residues R2, R3 and R4 of the general structure (I) denote a hydrogen atom.

4. The agent according to one of claims 1 to 3, **characterised in that** the physiologically tolerable anion $X^-$ denotes a halide ion, hydrogen sulfate, ½ sulfate, p-toluenesulfonate, benzenesulfonate or acetate.

5. The agent according to one of claims 1 to 4, **characterised in that** at least one cationic compound of the general structure (1) is included which is selected from compounds of the group formed from
N-methyl-3,4-dihydroisoquinoline p-toluenesulfonate,
N-methyl-3,4-dihydroisoquinoline benzenesulfonate,
N-methyl-3,4-dihydroisoquinoline hydrogen sulfate,
N-allyl-3,4-dihydroisoquinoline p-toluenesulfonate,
N-allyl-3,4-dihydroisoquinoline benzenesulfonate,
N-allyl-3,4-dihydroisoquinoline bromide,
N-allyl-3,4-dihydroisoquinoline acetate,
3,4-dihydro-2-(3-hydroxypropyl)isoquinoline p-toluenesulfonate,
3,4-dihydro-2-(3-hydroxypropyl)isoquinoline benzenesulfonate,
3,4-dihydro-2-(3-hydroxypropyl)isoquinoline bromide,
3,4-dihydro-2-(3-hydroxypropyl)isoquinoline acetate,
3,4-dihydro-2-(2-hydroxyethyl)isoquinoline p-toluenesulfonate,
3,4-dihydro-2-(2-hydroxyethyl)isoquinoline benzenesulfonate,
3,4-dihydro-2-(2-hydroxyethyl)isoquinoline bromide or
3,4-dihydro-2-(2-hydroxyethyl)isoquinoline acetate.

6. The agent according to one of claims 1 to 5, **characterised in that** n in the alkanol amines of the general formula (II) denotes a whole number from 2 to 4.

7. The agent according to one of claims 1 to 6, **characterised in that** the alkanol amine of the general formula (II) is a triethanolamine.

8. The agent according to one of claims 1 to 7, **characterised in that** the cationic 3,4-dihydroisoquinoline derivatives of the general structure (I) are included in an amount from 0.03 to 65.00 mmol, in particular 1.00 to 40.00 mmol, relative in each case to 100 g of the ready-to-use agent.

9. The agent according to one of claims 1 to 8, **characterised in that** the alkanol amines of the general structure (II) are included in an amount from 0.03 to 65.00 mmol, in particular 1.00 to 40.00 mmol, relative in each case to 100 g of the ready-to-use agent.

10. The agent according to one of claims 1 to 9, **characterised in that** it contains - relative to its weight - 0.5 to 12 wt. %, preferably 2 to 10 wt.% and particularly preferably 3 to 6 wt.% of hydrogen peroxide (calculated as 100% $H_2O_2$).

11. The agent according to one of claims 1 to 10, **characterised in that** the pH of the ready-to-use application mixture is between 7 and 11, in particular between 8 and 10.5.

12. The agent according to one of claims 1 to 11, **characterised in that** at least one inorganic persulfate salt is additionally included.

13. The agent according to claim 12, wherein the inorganic persulfate salts are selected from at least one compound from the group consisting of ammonium peroxydisulfate, potassium peroxydisulfate and sodium peroxydisulfate.

**14.** A method for bleaching keratin fibers **characterised in that** an agent according to one of claims 1 to 13 is applied to the keratinous fibers, left on the fibers for 5 to 60 minutes and then rinsed out again or washed out with a shampoo.

**15.** Cosmetic use of an agent according to one of claims 1 to 13 for bleaching keratinous fibers, in particular human hair.

**Revendications**

**1.** Agent pour l'éclaircissement de fibres kératiniques, **caractérisé en ce qu'**il contient, dans un support cosmétique approprié,

- au moins un dérivé cationique de 3,4-dihydro-isoquinoléinium présentant la structure générale suivante (I),

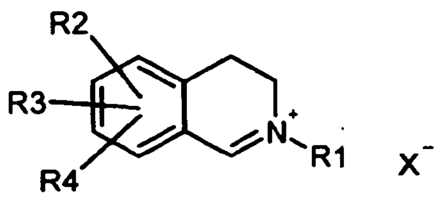

(I),

où

le radical R1 représente un groupe $C_1$-$C_6$-alkyle, $C_2$-$C_6$-alcényle, $C_2$-$C_6$-hydroxyalkyle, $C_1$-$C_6$-alcoxy-$C_2$-$C_6$-alkyle, carboxy-$C_1$-$C_6$-alkyle, aryl-$C_1$-$C_6$-alkyle, $C_1$-$C_6$-dialkylamino-$C_2$-$C_6$-alkyle, hétéroaryl-$C_1$-$C_6$-alkyle, 3-oxobutyle, 2-oxopropyle, aryle ou hétéroaryle,
les radicaux R2, R3 et R4 peuvent représenter, indépendamment l'un de l'autre, un atome d'hydrogène, un groupe hydroxy, un groupe amino, un groupe di($C_1$-$C_6$)alkylamino, un groupe $C_1$-$C_6$-alcoxy, halogène, un groupe nitro, un groupe carboxy, un groupe nitrile, un groupe aryle le cas échéant substitué, un groupe $C_2$-$C_6$-alcényle, un groupe hétéroaryle le cas échéant substitué ou R2 et R3 peuvent former, ensemble, un autre cycle condensé carbocyclique ou hétérocyclique, qui peut être saturé ou insaturé et le cas échéant substitué par jusqu'à trois substituants,
l'anion X- représente un anion physiologiquement acceptable,

- au moins une alcanolamine présentant la structure générale suivante (II),

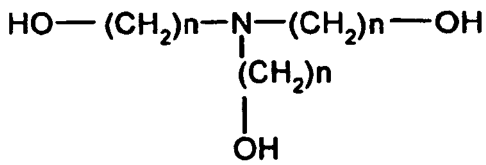

(II),

où

n représente un nombre naturel de 2 à 6, et

- du peroxyde d'hydrogène.

**2.** Agent selon la revendication 1, **caractérisé en ce que** le radical R1 de structure générale (I) représente un groupe $C_1$-$C_6$-alkyle, un groupe $C_2$-$C_6$-alcényle ou un groupe $C_2$-$C_6$-hydroxyalkyle.

**3.** Agent selon les revendications 1 et 2, **caractérisé en ce que** les radicaux R2, R3 et R4 de structure générale (I) représentent un atome d'hydrogène.

**4.** Agent selon l'une quelconque des revendications 1 à 3, **caractérisé en ce que** l'anion X- physiologiquement acceptable représente un ion halogénure, hydrogénosulfate, ½-sulfate, p-toluènesulfonate, benzènesulfonate ou acétate.

5. Agent selon l'une quelconque des revendications 1 à 4, **caractérisé en ce qu'**il contient au moins un composé cationique de structure générale (I) choisi parmi les composés du groupe formé par
le p-toluènesulfonate de N-méthyl-3,4-dihydro-isoquinoléinium,
le benzènesulfonate de N-méthyl-3,4-dihydro-isoquinoléinium
l'hydrogénosulfate de N-méthyl-3,4-dihydro-isoquinoléinium
le p-toluènesulfonate de N-allyl-3,4-dihydro-isoquinoléinium
le benzènesulfonate de N-allyl-3,4-dihydro-isoquinoléinium
le bromure de N-allyl-3,4-dihydro-isoquinoléinium
l'acétate de N-allyl-3,4-dihydro-isoquinoléinium
le p-toluènesulfonate de 3,4-dihydro-2-(3-hydroxypropyl)isoquinoléinium
le benzènesulfonate de 3,4-dihydro-2-(3-hydroxypropyl)isoquinoléinium
le bromure de 3,4-dihydro-2-(3-hydroxypropyl)isoquinoléinium
l'acétate de 3,4-dihydro-2-(3-hydroxypropyl)isoquinoléinium
le p-toluènesulfonate de 3,4-dihydro-2-(2-hydroxyéthyl)isoquinoléinium
le benzènesulfonate de 3,4-dihydro-2-(2-hydroxyéthyl)isoquinoléinium
le bromure de 3,4-dihydro-2-(2-hydroxyéthyl)isoquinoléinium ou
l'acétate de 3,4-dihydro-2-(2-hydroxyéthyl)isoquinoléinium

6. Agent selon l'une quelconque des revendications 1 à 5, **caractérisé en ce que** n dans les alcanolamines de formule générale (II) représente un nombre entier de 2 à 4.

7. Agent selon l'une quelconque des revendications 1 à 6, **caractérisé en ce qu'**il s'agit, pour l'alcanolamine de formule générale (II), de triéthanolamine.

8. Agent selon l'une quelconque des revendications 1 à 7, **caractérisé en ce que** les dérivés cationiques de 3,4-dihydro-isoquinoléinium de structure générale (I) sont contenus en une quantité de 0,03 à 65,00 mmoles, en particulier de 1,00 à 40,00 mmoles, à chaque fois par rapport à 100 g de l'agent prêt à l'emploi.

9. Agent selon l'une quelconque des revendications 1 à 8, **caractérisé en ce que** les alcanolamines de structure générale (II) sont contenues en une quantité de 0,03 à 65,00 mmoles, en particulier de 1,00 à 40,00 mmoles, à chaque fois par rapport à 100 g de l'agent prêt à l'emploi.

10. Agent selon l'une quelconque des revendications 1 à 9, **caractérisé en ce qu'**il contient - par rapport à son poids, 0,5 à 12% en poids, de préférence de 2 à 10% en poids et en particulier de préférence 3 à 6% en poids de peroxyde d'hydrogène (calculé sous forme de $H_2O_2$ à 100%).

11. Agent selon l'une quelconque des revendications 1 à 10, **caractérisé en ce que** le pH du mélange d'utilisation prêt à l'emploi se situe entre 7 et 11, en particulier entre 8 et 10,5.

12. Agent selon l'une quelconque des revendications 1 à 11, **caractérisé en ce qu'**il contient en outre au moins un sel de persulfate inorganique.

13. Agent selon la revendication 12, **caractérisé en ce que** les sels de persulfate inorganiques sont choisis parmi au moins un composé du groupe constitué par le peroxydisulfate d'ammonium, le peroxydisulfate de potassium et le peroxydisulfate de sodium.

14. Procédé pour l'éclaircissement de fibres kératiniques, **caractérisé en ce qu'**on applique un agent selon l'une quelconque des revendications 1 à 13 sur les fibres kératiniques, on le laisse agir pendant 5 à 60 minutes sur les fibres, puis on l'élimine à nouveau par rinçage ou par lavage avec un shampooing.

15. Utilisation cosmétique d'un agent selon l'une quelconque des revendications 1 à 13 pour l'éclaircissement de fibres kératiniques, en particulier de cheveux humains.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 20050120447 A1
- EP 1905418 A1
- EP 1169423 B1
- EP 116924 B1
- WO 9823717 A1
- US 5785886 A
- US 5576282 A
- WO 2009043613 A
- EP 998908 A2


### In der Beschreibung aufgeführte Nicht-Patentliteratur


- **VON KH. SCHRADER.** Grundlagen und Rezepturen der Kosmetika. 1989
- Kosmetik. Georg Thieme Verlag, 1995